(19) Europäisches Patentamt
European Patent Office
Office européen des brevets

(11) Publication number: 0 388 660 A2

(12) # EUROPEAN PATENT APPLICATION

(21) Application number: 90103595.6

(22) Date of filing: 23.02.90

(51) Int. Cl.[5]: C07D 498/10, C07D 498/20, G03C 1/73, //(C07D498/10, 265:00,221:00),(C07D498/20, 265:00,221:00,221:00)

(30) Priority: 23.02.89 JP 44132/89
16.06.89 JP 153897/89

(43) Date of publication of application: 26.09.90 Bulletin 90/39

(84) Designated Contracting States: DE FR GB IT

(71) Applicant: SHOWA DENKO KABUSHIKI KAISHA
13-9, Shiba Daimon 1-chome
Minato-ku, Tokyo 105(JP)

(72) Inventor: Yamashina, Naoko
3-2, Chidori-cho, Kawasaki-ku
Kawasaki-shi, Kanagawa(JP)
Inventor: Kawauchi, Susumu
3-2, Chidori-cho, Kawasaki-ku
Kawasaki-shi, Kanagawa(JP)
Inventor: Ohira, Manabu
3-2, Chidori-cho, Kawasaki-ku
Kawasaki-shi, Kanagawa(JP)

(74) Representative: Strehl, Schübel-Hopf, Groening
Maximilianstrasse 54 Postfach 22 14 55
D-8000 München 22(DE)

(54) **Photochromic compound.**

(57) Disclosed is a photochromic compound of the formula (I):

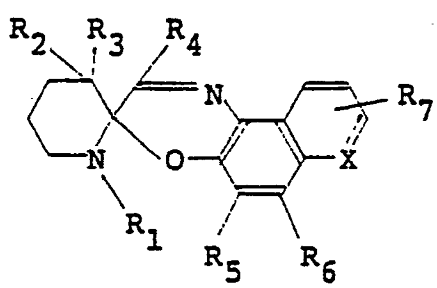

(I)

wherein (a) X is C or N, (b) $R_1$ is a $C_{1-18}$ alkyl, aralkyl having $C_{1-5}$ alkyl, $C_{2-10}$ alkoxyalkyl, $C_{2-10}$ alkenyl, phenyl, benzyl, substituted phenyl or substituted benzyl, (c) $R_2$ and $R_3$ are a group as defined above with respect to $R_1$, H, $C_{5-10}$ alicyclic, norbornyl or adamantyl, (d) $R_4$ is H, $C_{1-18}$ alkyl, aralkyl having $C_{1-5}$ alkyl, $C_{1-18}$ heteroalkyl or $C_{2-10}$ alkenyl, (e) $R_5$ and $R_7$ are H, $C_{1-9}$ alkyl, $C_{1-5}$ alkoxy, halo, nitro or cyano, and (f) $R_6$ is H, $C_{1-10}$ alkylamino, phenylamino, benzylamino, substituted phenylamino, substituted benzylamino or $C_{4-12}$ cyclic amino, with the proviso that when X is C, $R_6$ is not H.

EP 0 388 660 A2

# PHOTOCHROMIC COMPOUND

## BACKGROUND OF THE INVENTION

### (1) Field of the Invention

This invention relates to a novel photochromic compound. More particularly, it relates to a photochromic compound which assumes a red, pink or violet color when irradiated with ultraviolet rays and has an excellent heat resistance and good repeatability.

### (2) Description of the Related Art

By the term "photochromism" is meant the reversible phenomenon in which a change of color is caused by an irradiation with a light containing ultraviolet rays, such as sunlight or the light of a mercury lamp, and the original color is restored when the irradiation is stopped and the object is stood in a dark place. A compound showing this phenomenon is called a photochromic compound.

Among the organic photochromic compounds, spiro-oxazine compounds have a good repeatability. For example, 1,3,3-trimethylspiro(indoline-2,3′-3H-naphtho[2,1-b]-1,4-oxazine) and its derivatives are disclosed in Japanese Examined Patent Publication No. 45-28892, Japanese Examined Patent Publication No. 49-48631, Japanese Unexamined Patent Publication No. 55-36284 and Japanese Unexamined Patent Publication No. 60-112880. Most of these compounds are tinted with a blue color when irradiated with ultraviolet rays, and thus have a limited usage. Practically, variations of colors are desired, and Japanese Unexamined Patent Publication No. 62-145089 discloses a compound having an absorption in a shorter wavelength region than the foregoing compounds. Nevertheless, the melting point of this compound is low, and thus this compound has a poor heat resistance is poor.

## SUMMARY OF THE INVENTION

A primary object of the present invention is to provide a photochromic compound which is tinted with a pink, violet or red color when irradiated with ultraviolet rays and has an excellent heat resistance and a good repeatability.

The photochromic compound of the present invention is represented by the following formula (I):

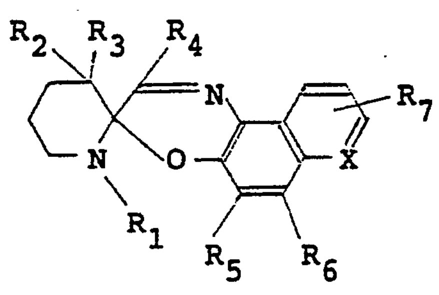

(I)

wherein (a) X represents carbon or nitrogen, (b) $R_1$ represents a linear or branched alkyl group having 1 to 18 carbon atoms, an aralkyl group having a linear or branched alkyl group having 1 to 5 carbon atoms, an alkoxyalkyl group having 2 to 10 carbon atoms, an alkenyl group having 2 to 10 carbon atoms, a phenyl group, a benzyl group, a mono- or di-substituted phenyl group, or a mono- or di-substituted benzyl group, (c) $R_2$ and $R_3$ independently represent a group as defined above with respect to $R_1$, a hydrogen atom, an alicyclic ring having 5 to 10 carbon atoms, which is bonded between groups present on the same skeleton carbon atom or between groups present on adjacent skeleton carbon atoms (ortho positions), a norbornyl group or an adamantyl group, (d) $R_4$ represents a hydrogen atom, a linear or branched alkyl group having 1 to 18 carbon atoms, an aralkyl group having a linear or branched alkyl group having 1 to 5 carbon atoms, a linear or branched heteroalkyl group having 1 to 18 carbon atoms, or an alkenyl group having 2 to 10 carbon atoms, (e) $R_5$ and $R_7$ independently represent a hydrogen atom, a linear or branched alkyl group

having 1 to 9 carbon atoms, an alkoxy group having 1 to 5 carbon atoms, a halogen atom, a nitro group or a cyano group, and (f) $R_6$ represents a hydrogen atom, a linear or branched mono- or di-alkylamino group having 1 to 10 carbon atoms, a phenylamino group, a benzylamino group, a mono- or di-substituted phenylamino group, a mono- or di-substituted benzylamino group, or a cyclic amino group having 4 to 12 carbon atoms and up to 3 hetero atoms, with the proviso that when X is carbon, $R_6$ is not a hydrogen.

BRIEF DESCRIPTION OF THE DRAWINGS

Figure 1 shows the $H^1$-NMR spectrum of the compound of Example 1;
Fig. 2 shows the $C^{13}$-NMR spectrum of the compound of Example 1;
Fig. 3 shows the IR spectrum of the compound of Example 1;
Fig. 4 shows the visible ray absorption spectrum of the compound of Example 1;
Fig. 5 shows the $H^1$-NMR spectrum of the compound of Example 3;
Fig. 6 shows the $C^{13}$-NMR spectrum of the compound of Example 3;
Fig. 7 shows the IR spectrum of the compound of Example 3; and
Fig. 8 shows the visible ray absorption spectrum of the compound of Example 3.

DESCRIPTION OF THE PREFERRED EMBODIMENTS

Among the photochromic compounds represented by the above-mentioned general formula (I), photochromic compounds in which $R_1$, $R_2$ and $R_3$ represent a linear or branched alkyl group having 1 to 18 carbon atoms, $R_4$ represents a hydrogen atom, $R_5$ represents a hydrogen atom or an alkoxy group having 1 to 5 carbon atoms, $R_6$ represents a cyclic amino group having 4 to 12 carbon atoms and up to 3 hetero atoms and $R_7$ represents a hydrogen atom or an alkoxy group having 1 to 5 carbon atoms are preferable. Photochromic compounds in which $R_1$ , $R_2$ and $R_3$ represent a methyl group, $R_4$ represents a hydrogen atom, $R_5$ represents a hydrogen atom or a methoxy group, $R_6$ represents a piperidino group, a pyrrolidino group or a morpholino group and $R_7$ represents a hydrogen atom or a methoxy group are more preferable. Of these preferable photochromic compounds, a compound in which X is nitrogen is tinted with a red color, and a compound in which X is carbon is tinted with a pink or violet color.

As typical examples of the photochromic compound (spironaphtho-oxazine compound) in which X is nitrogen, there can be mentioned 1′,3′,3′-trimethylspiro(piperidine-3,2′-2H-pyrido[3,2-f]-1,4-benzo-oxazine), 6-morpholino-1′,3′,3′-trimethylspiro(piperidine-3,2′-2H-pyrido[3,2-f]-1,4-benzo-oxazine), 6-piperidino-1′,3′,3′-trimethylspiro(piperidine-3,2′-2H-pyrido[3,2-f]-1,4-benzo-oxazine) and 6-pyrrolidino-1′,3′,3′-trimethylspiro-(piperidine-3,2′-2H-pyrido[3,2-f]-1,4-benzo-oxazine).

As typical examples of the photochromic compound (spironaphtho-oxazine compound) in which X is carbon, there can be mentioned 6-piperidino-1′,3′,3′-trimethylspiro(3H-naphtho[2,1-b]-1,4-oxazine-3,2′-piperidine), 6-morpholino-1′,3′,3′-trimethylspiro(3H-naphtho[2,1-b]-1,4-oxazine-3,2′-piperidine), 9-methoxy-6-piperidino-1′,3′,3′-trimethylspiro(3H-naphtho[2,1-b]-1,4-oxazine-3,2′-piperidine), 9-methoxy-6-morpholino-1′,3′,3′-trimethylspiro(3H-naphtho[2,1-b]-1,4-oxazine-3,2′-piperidine), 5-methoxy-6-piperidino-1′,3′,3′-trimethylspiro(3H-naphtho[2,1-b]-1,4-oxazine-3,2′-piperidine) and 5-methoxy-6-morpholino-1′,3′,3′-trimethylspiro(3H-naphtho[2,1-b]-1,4-oxazine-3,2′-piperidine).

The photochromic compounds of the present invention are novel compounds not disclosed in any literature reference. Among these photochromic compounds, those in which X is nitrogen can be prepared, for example, according to the following process.

A 2-methylene-piperidine derivative represented by the following general formula (II):

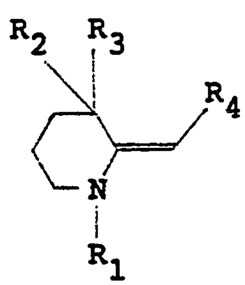

(II)

wherein $R_1$ , $R_2$ , $R_3$ and $R_4$ are as defined above, and a 5-nitroso-6-quinolinol derivative represented by

the following general formula (III):

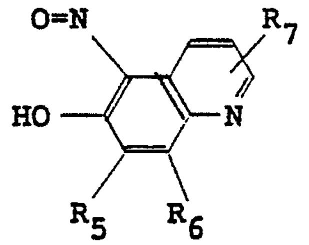

(III)

wherein $R_5$ , $R_6$ and $R_7$ are as defined above, are heat-reacted in an appropriate organic solvent such as ethanol, toluene or trichlene preferably at 30 to 60° C, especially at a temperature of up to the boiling point of the solvent used. The molar ratio of the compound of formula (III) used to the compound of formula (II) used is at least 1, preferably 1 to 3.

Alternatively, instead of the 2-methylene-piperidine derivative of formula (II) used in the above-mentioned process, a 3,4,5,6-tetrahydropyridinium salt derivative represented by the following formula (IV):

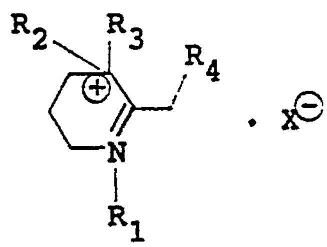

(IV)

wherein $R_1$ , $R_2$ , $R_3$ and $R_4$ are as defined above,
and X⊖ represents an anion such as an iodine ion, a bromine ion or a tosylate ion,
is used and an amine is used in an amount of at least 1 mole, preferably 1 to 3 moles, per mole of the 3,4,5,6-tetrahydropyridinium salt derivative. These compounds are reacted with the 5-nitroso-6-quinolinol derivative of formula (III) in the same manner as described above to prepare the intended photochromic compound. As the amine, there can be mentioned, for example, triethylamine, piperidine, pyrrolidine and morpholine.

The photochromic compounds in which X is carbon can be prepared, for example, according to the following process.

Namely, these photochromic compounds can be prepared in the same manner as adopted for the preparation of the above-mentioned compounds in which X is nitrogen, except that instead of the 5-nitroso-6-quinolino derivative of formula (III), a 1-nitroso-2-naphthol derivative represented by the following general formula (V):

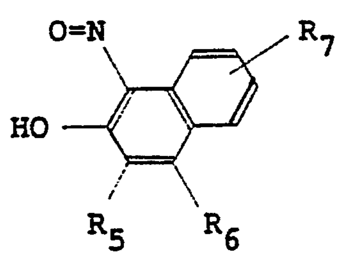

(V)

wherein $R_5$ , $R_6$ and $R_7$ are as defined above,
is used at a molar ratio of at least 1, preferably 1 to 3.

Alternatively, instead of the 2-methylene-piperidine derivative of formula (II) to be reacted with the 1-nitroso-2-napthol of formula (V), a corresponding tetrahydropyridinium salt compound and an amine are used, and the reaction is carried out in the same manner as described above. As the amine, there can be mentioned, for example, triethylamine, piperidine, pyrrolidine and morpholine. The amount of the amine

used is at least 1 mole, preferably 1 to 3 moles, per mole of the tetrahydropyridinium salt.

If desired, the prepared photochromic compound can be purified to a refined product by an active carbon treatment, a recrystallization treatment or a column separation treatment.

The photochromic compound of the present invention can be dissolved in usual organic solvents such as benzene, toluene, chloroform, ethyl acetate, methyl ethyl ketone, acetone, ethanol, methanol, isopropyl alcohol, n-butyl alcohol, benzyl alcohol, tetrahydrofuran, dimethylformamide, dimethylsulfoxide, dioxane, acetonitrile, methylcellosolve and ethylene glycol. Each of the obtained solutions shows such a photochromism that it is colorless in the dark, but when irradiated with ultraviolet rays, is tinted with a red, pink or violet color.

The photochromic compound of the present invention is soluble in a colorless or transparent solution prepared from a transparent polymer or copolymer or a transparent mixture thereof and an appropriate solvent, for example, in a transparent host material, as described hereinafter, which is dissolved in at least one member selected from the above-mentioned organic solvent. As examples of the solution, there can be mentioned a polyvinyl acetate/acetone solution, a nitrocellulose/acetonitrile solution, a polyvinyl chloride/methyl ethyl ketone solution, a polymethyl methacrylate/acetone solution, an acetylcellulose/dimethylformamide solution, a polyvinyl pyrrolidone/acetonitrile solution, a polystyrene/toluene solution and an ethylcellulose/methylene chloride solution.

If the above photochromic solution or composition is coated and dried on a transparent support such as triacetylcellulose or polyethylene terephthalate or baryta paper, a photochromic material which is tinted with a red, pink or violet color when irradiated with ultraviolet rays and becomes colorless again when the irradiation with ultraviolet rays is stopped, can be obtained.

This photochromic material can be prepared by known procedures. Namely, the photochromic compound of general formula (I) is dissolved in, kneaded with, or coated on a host material such as a resin, an oil or a paper, and is thus incorporated into a material of an optional shape and state, for example, a solid material such as a film, a lens or a plate, or a liquid material such as an oil or an emulsion. The amount of the photochromic compound of the present invention incorporated in a material as described above is not particularly critical, but the photochromic compound is usually incorporated in an amount of $10^{-5}$ to 50% by weight, preferably in an amount of $10^{-4}$ to 20% by weight.

The photochromic compound of the present invention can be advantageously used for the manufacture of, for example, plastic sun glasses capable of automatically adjusting the quantity of light admitted therethrough, skiing goggles, sun visors, window panes, glass laminates, packaging materials, accessories, decorations, automobile trims, paints, inks, manicures, cosmetics, memory materials, and actinometers.

The present invention will now be described in detail with reference to the following examples, that by no means limit the technical scope of the invention.

## Example 1

### 1′,3′,3′-Trimethylspiro(piperidine-3,2′-2H-pyrido[3,2-f]-1,4-benzo-oxazine)

A mixture formed by adding 30 g of 5-nitroso-6-quinolinol to 150 ml of trichlene was heated at 65°C to dissolve the quinolinol. The mixture was stirred for 30 minutes, and a mixture comprising 23.5 g of 1,3,3-trimethyl-2-methylene-piperidine and 40 ml of trichlene was added to the above mixture over a period of 15 minutes. Then the mixture was further stirred at 65°C for 3 hours.

Activated carbon was added to the reaction mixture and the mixture was filtered. Trichlene was substantially completely removed from the obtained filtrate under a reduced pressure, ethanol was added to the residue, and the mixture was allowed to stand in the cold place for about 3 days. The precipitated crystal was recovered by filtration, the crude crystal was dissolved in acetone in an amount 5 times the amount of the crude crystal, activated carbon was added to the solution, and the mixture was heated. The mixture was filtered, the filtrate was concentrated, the obtained solution was allowed to stand in a cold place, and the precipitated crystal was recovered by filtration, washed with a small amount of cold alcohol, and dried to obtain 1.0 g of the intended compound (greyish white crystal). The physical properties of the obtained compound were as shown below.

Melting point
104 to 106°C
Elementary analysis values
calculated: C = 73.19%, H = 7.17%, N = 14.23%

found: C = 73.87%, H = 7.54%, N = 13.51%

$H^1$-NMR

The measurement was conducted in deuterated chloroform as the solvent by using TMS (tetramethylsilane) as the internal standard and a spectrometer JEOL FX-200. The obtained $H^1$-NMR spectrum is shown in Fig. 1.

$C^{13}$-NMR

The measurement was conducted in deuterated chloroform as the solvent by using a spectrometer JEOL FX-200. The obtained $C^{13}$-NMR spectrum is shown in Fig. 2.

IR-spectrum

The measurement was conducted by using an infrared spectrophotometer Diglab FTS 15E/D. The obtained IR-spectrum is shown in Fig. 3.

Visible ray absorption spectrum

The change of the visible ray absorption spectrum in a methanol solution before and after irradiation with ultraviolet rays was measured by an ultraviolet-visible spectrophotometer (MCPD-1000 supplied by Otsuka Denshi). The concentration was $5 \times 10^{-5}$ mole/ℓ and the measurement temperature was 25 °C. The obtained visible ray absorption spectrum is shown in Table 4.

Decolorization speed

The decolorization speed and half-value period were determined from the change with time of the absorbance at the visible ray absorption maximum wavelength after irradiation with ultraviolet rays. The decolorization speed at 25 °C in a methanol solution having a concentration of $5 \times 10^{-5}$ mole/ℓ was $7.45 \times 10^{-4}$ $sec^{-1}$, and the half-value period was 930.7 seconds.

Repetition durability

After coloration-decolorization was repeated 1,000 times by suing an XeCl excimer laser, the coloring capacity was examined and it was found that the coloring capacity was 92% of the initial capacity.

Example 2

1′,3′,3′ Trimethylspiro(piperidine-3,2′-2H-pyrido[3,2-f]-1,4-benzo-oxazine

A mixture obtained by adding 30 g of 5-nitoso-6-quinolinol and 35 g of triethylamine into 150 ml of trichlene was heated at 65 °C to form a solution. Then, the mixture was stirred for 30 minutes, and a mixture (slurry) comprising 47.2 g of 3,4,5,6-tetrahydro-1,2,3,3-tetramethylpyridinium iodide and 40 ml of trichlene was added to the above mixture over a period of 15 minutes. The mixture was further stirred at 65 °C for 3 hours.

Activated carbon was added to the reaction mixture, and the mixture was filtered. Trichlene was substantially completely removed from the filtrate under a reduced pressure, ethanol was added to the residue, and the mixture was allowed to stand in a cold place for about 3 days. The precipitated crystal was recovered by filtration, and the obtained crude crystal was dissolved in acetone in an amount 5 times the

amount of the crude crystal. Activated carbon was added to the solution, and the mixture was heated and filtered. The filtrate was concentrated and the obtained solution was allowed to stand in a cold place, and the precipitated crystal was recovered by filtration, washed with a small amount of cold alcohol and dried to obtain 1.5 g of the intended compound (greyish white crystal). The physical properties of the obtained compound were similar to those of the compound of Example 1.

Example 3

6-Piperidino-1′,3′,3′-trimethylspiro(3H-naphtho[2,1-b]-1,4-oxazine-3,2′-piperidine)

A mixture obtained by adding 30 g of 1-nitroso-2-naphthol and 30 g of piperidine into 150 ml of trichlene was heated at 65° C to form a solution. The mixture was stirred for 30 minutes, and a mixture comprising 23.5 g of 1,3,3-trimethyl-2-methylenepiperidine and 40 ml of trichlene was added to the above mixture over a period of 15 minutes. The mixture was further stirred at 65° C for 3 hours.

Activated carbon was added to the reaction mixture, and the mixture was filtered. Trichlene was substantially completely removed from the filtrate under a reduced pressure, and ethanol was added to the residue and the mixture was allowed to stand in a cold place for about 3 days. The precipitated crystal was recovered by filtration and dissolved in acetone in an amount 5 times the amount of the crystal. Activated carbon was added to the solution, and the mixture was heated and filtered. The filtrate was concentrated, and the obtained solution was allowed to stand in a cold place. The precipitated crystal was recovered by filtration, washed with a small amount of cold alcohol and dried to obtain 7.5 g of the intended compound (greyish white crystal). The physical properties of the obtained compound were as shown below.

Melting point
175 to 176° C
Elementary analysis values
calculated: C = 76.35%, H = 8.28%, N = 11.13%
found: C = 76.16%, H = 8.44%, N = 11.16%

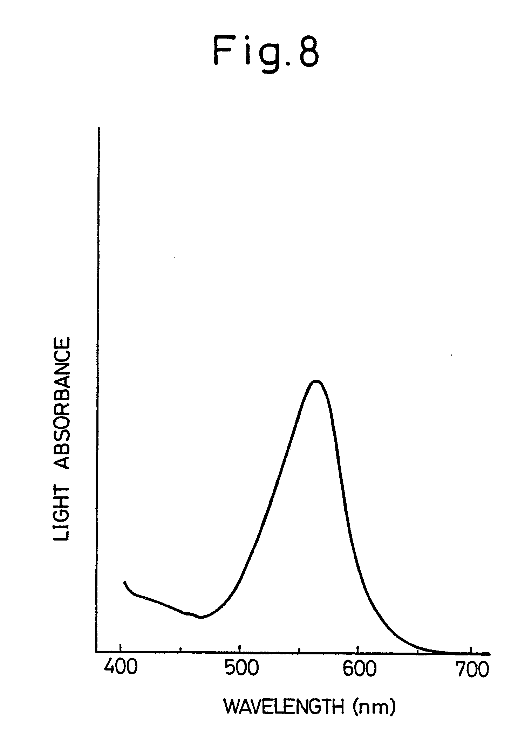

The $H^1$-NMR, $C^{13}$-NMR, IR and visible ray absorption spectra of the obtained compound, determined in the same manner as described in Example 1, are shown in Figs. 5, 6, 7 and 8, respectively.

The decolorization speed and half-value period were measured in the same manner as described in Example 1, and it was found that the decolorization speed at 25° C in a methanol solution having a concentration of $5 \times 10^{-5}$ mole/ℓ was 1.33 $sec^{-1}$, and the half-value period was 0.5 second. When the repetition durability was examined in the same manner as described in Example 1, and it was found that 92% of the initial coloring capacity was retained.

Example 4

6-Piperidino-1′,3′,3′-trimethylspiro(3H-naphtho[2,1-b]-1,4-oxazine-3,2′-piperidine

A mixture obtained by adding 30 g of 1-nitroso-2-naphthol and 45 g of piperidine into 150 ml of trichlene was heated at 65° C to form a solution. The mixture was stirred for 30 minutes, and a mixture (slurry) comprising 47.2 g of 3,4,5,6-tetrahydro-1,2,3,3-tetramethylpyridinium iodide and 40 ml of trichlene was added to the above mixture over a period of 15 minutes and the resulting mixture was further stirred at 65° C for 3 hours.

Activated carbon was added to the reaction mixture, and the mixture was filtered. Trichlene was substantially completely removed from the filtrate under a reduced pressure and ethanol was added to the residue. The mixture was allowed to stand in a cold place for about 3 days, and the precipitated crystal was recovered by filtration and dissolved in acetone in an amount 5 times the amount of the crude crystal. Activated carbon was added to the solution and the mixture was heated and filtered. The filtrate was concentrated and the obtained solution was allowed to stand in a cold place. The precipitated crystal was recovered by filtration, washed with a small amount of cold alcohol and dried to obtain 5.8 g of the intended compound (greyish white crystal). The physical properties of the obtained compound were similar to those of the compound of Example 3.

Example 5

6-Morpholino-1′,3′,3′-trimethylspiro(3H-naphtho[2,1-b]-1,4-oxadine-3,2′-piperidine

A mixture obtained by adding 30 g of 1-nitroso-2-naphthol and 30 g of morpholine into 150 ml of trichlene was heated at 65° C to form a solution, and the mixture was stirred for 30 minutes. A mixture comprising 23.5 g of 1,3,3-trimethyl-2-methylene-piperidine and 40 ml of trichlene was added to the above mixture over a period of 15 minutes and the mixture was further stirred at 65° C for 3 hours.

Activated carbon was added to the reaction mixture, and the mixture was filtered. Trichlene was substantially completely removed from the filtrate under a reduced pressure, and ethanol was added to the residue and the mixture was allowed to stand in a cold place for about 3 hours. The precipitated crystal was recovered by filtration and the crude crystal was dissolved in acetone in an amount 5 times the amount of the crystal, and activated carbon was added to the solution and the mixture was filtered. The filtrate was concentrated and the obtained solution was allowed to stand in a cold place. The precipitated crystal was recovered by filtration, washed with a small amount of cold water and dried to obtain 5.5 g of the intended compound (greyish white crystal).

The physical properties of the obtained compound were as shown below.

Melting point

157 to 159° C

Elementary analysis values

calculated: C = 72.79%, H = 7.70%, N = 11.07%

found: C = 72.64%, H = 7.52%, N = 11.08%

$H^1$-NMR

The measurement was carried out in the same manner as described in Example 1. The obtained results were as shown below.

$\delta_H$ (ppm): 0.88 (s, 3′-Me, 3H), 1.20 (s, 4′-H, 1H), 1.25 (s, 3′-Me, 3H), 1.60 (m, 5′-H, 1H), 1.91 (m, 5′-H, 1H), 1.99 (m, 4′-H, 1H), 2.24 (s, 1′-Me, 3H), 2,60 (m, 6′-H, 1H), 2.87 (m, 6′-H, 1H), 3.33 (5, morpholine-N-(CH2)2, 4H), 3.98 (t, morpholine-O-(CH2)2, 4H), 6.68 (s, 5-H, 1H), 7.35 (m, 8-H, 1H), 7.43 (s, 2-H, 1H), 7.51 (m, 9-H, 1H), 8.03 (d, 7-H, 1H), 8.50 (d, 10-H, 1H)

$C^{13}$-NMR

The measurement was carried out in the same manner as described in Example 1. The results were as shown below.

$\delta_C$ (ppm): 21.0 (5′-C), 22.1 (3′-Me), 26.5 (3′-Me), 32.8 (4′-C), 39.5 (N-Me), 39.9 (Me2-C), 48.2 (6′-C), 53.5 (morpholine-NC2), 67.3 (morpholine-OC2), 92.7 (2′-C), 105.5 (5-C), 119.0 (10b-C), 121. (10-C), 123.1 (8-C), 123.3 (7-C), 123.9 (6a-C), 126.9 (9-C), 132.2 (10a-C), 146.0 (4a-C), 151.1 (2-C), 151.3 (6-C)

Visible ray absorption spectrum

The measurement was carried out in the same manner as described in Example 1. The visible ray absorption maximum wavelength in the methanol solution was 564 nm.

Decolorization speed

The decolorization speed in a methanol solution having a concentration of 5 x $10^{-5}$ mole/ℓ at 25° C was 0.66 $sec^{-1}$, and the half-value period was 1.0 second.

Repetition durability

The repetition durability was examined in the same manner as described in Example 1, and it was found that 90% of the initial coloring capacity was retained.

## Claims

1. A photochromic compound represented by the following formula (I):

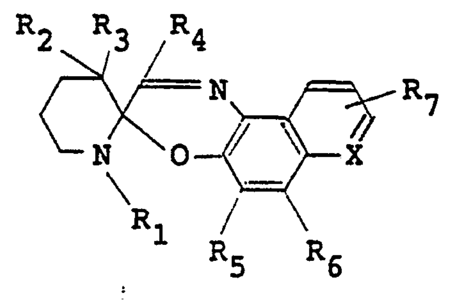

(I)

wherein (a) X represents carbon or nitrogen, (b) $R_1$ represents a linear or branched alkyl group having 1 to 18 carbon atoms, an aralkyl group having a linear or branched alkyl group having 1 to 5 carbon atoms, an alkoxyalkyl group having 2 to 10 carbon atoms, an alkenyl group having 2 to 10 carbon atoms, a phenyl group, a benzyl group, a mono- or di-substituted phenyl group, or a mono- or di-substituted benzyl group, (c) $R_2$ and $R_3$ independently represent a group as defined above with respect to $R_1$ , a hydrogen atom, an alicyclic ring having 5 to 10 carbon atoms, which is bonded between groups present on the same skeleton carbon atom or between groups present on adjacent skeleton carbon atoms (ortho positions), a norbornyl group or an adamantyl group, (d) $R_4$ represents a hydrogen atom, a linear or branched alkyl group having 1 to 18 carbon atoms, an aralkyl group having a linear or branched alkyl group having 1 to 5 carbon atoms, a linear or branched heteroalkyl group having 1 to 18 carbon atoms, or an alkenyl group having 2 to 10 carbon atoms, (e) $R_5$ and $R_7$ independently represent a hydrogen atom, a linear or branched alkyl group having 1 to 9 carbon atoms, an alkoxy group having 1 to 5 carbon atoms, a halogen atom, a nitro group or a cyano group, and (f) $R_6$ represents a hydrogen atom, a linear or branched mono- or di-alkylamino group having 1 to 10 carbon atoms, a phenylamino group, a benzylamino group, a mono- or di-substituted phenylamino group, a mono- or di-substituted benzylamino group, or a cyclic amino group having 4 to 12 carbon atoms and up to 3 hetero atoms, with the proviso that when X is carbon, $R_6$ is not a hydrogen.

2. A photochromic compound according to claim 1, wherein X is carbon or nitrogen, $R_1$ , $R_2$ and $R_3$ represent a linear or branched alkyl group having 1 to 18 carbon atoms, $R_4$ is a hydrogen atom, $R_5$ represents a hydrogen atom or an alkoxy group having 1 to 5 carbon atoms, $R_6$ represents a cyclic amino group having 4 to 12 carbon atoms and up to 3 hetero atoms, and $R_7$ represents a hydrogen atom or an alkoxy group having 1 to 5 carbon atoms.

3. A photochromic compound according to claim 1, wherein X is carbon or nitrogen, $R_1$ , $R_2$ and $R_3$ represent a methyl group, $R_4$ is a hydrogen atom, $R_5$ represents a hydrogen atom or a methoxy group, $R_6$ represents a piperidino group, a pyrrolidino group or a morpholino group, and $R_7$ represents a hydrogen atom or a methoxy group.

4. A process for the preparation of a photochromic compound represented by the following general formula (I):

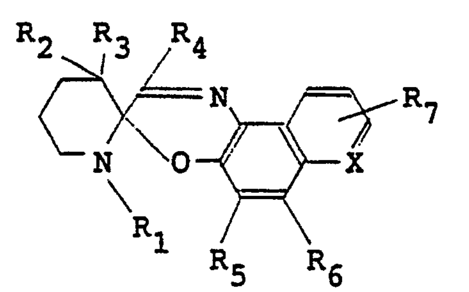

(I)

wherein X and $R_1$ through $R_7$ are as defined in claim 1,
which comprises heating and reacting a 2-methylene-piperidine derivative represented by the following

general formula (II):

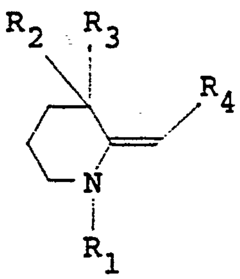

(II)

wherein $R_1$ , $R_2$ , $R_3$ and $R_4$ are as defined in claim 1,
with at least an equimolar amount of a 5-nitroso-6-quinolinol derivative represented by the following general formula (III):

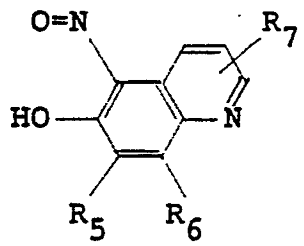

(III)

wherein $R_5$ , $R_6$ and $R_7$ are as defined in claim 1,
or with at least an equimolar amount of a 1-nitro-2-naphthol derivative represented by the following general formula (V):

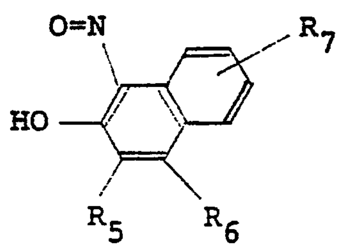

(V)

wherein $R_5$ , $R_6$ and $R_7$ are as defined in claim 1,
in an organic solvent.

5. A process for the preparation of a photochromic compound represented by the following general formula (I):

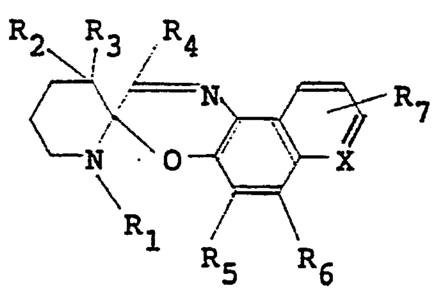

(I)

wherein X and $R_1$ through $R_7$ are as defined in claim 1,
which comprises heating and reacting a 3,4,5,6-tetrahydropyridinium salt derivative represented by the following general formula (IV):

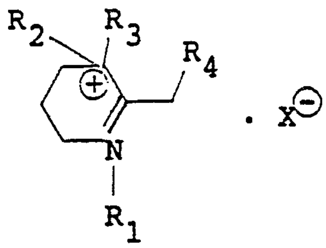

(IV)

wherein $R_1$ , $R_2$ , $R_3$ and $R_4$ are as defined in claim 1, and X⊖ represents an anion, with at least an equimolar amount of a 5-nitroso-6-quinolinol derivative represented by the following general formula (III):

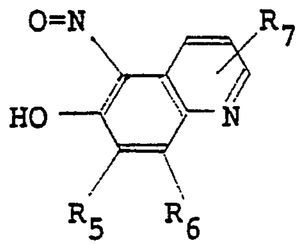

(III)

wherein $R_5$ , $R_6$ and $R_7$ are as defined in claim 1, or with at least an equimolar amount of a 1-nitroso-2-naphthol derivative represented by the following general formula (V):

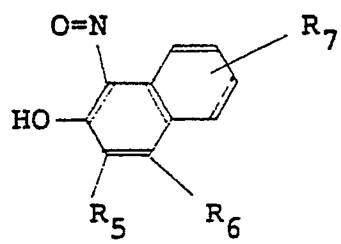

(V)

wherein $R_5$ , $R_6$ and $R_7$ are as defined in claim 1, in the presence of at least 1 mole, per mole of the tetrahydropyridinium salt derivative of formula (IV), of an amine in an organic solvent.

6. The use of a photochromic compound according to any of the claims 1 to 3 for the production of sun glasses and sun visors.

7. The use of a photochromic compound according to any of the claims 1 to 3 for the production of window panes or glass laminates.

8. The use of a photochromic compound according to any of the claims 1 to 3 for the production of paints.

9. The use of a photochromic compound according to any of the claims 1 to 3 for the production of memory materials.

10. The use of a photochromic compound according to any of the claims 1 to 3 for the production of actinometers.

Fig.1

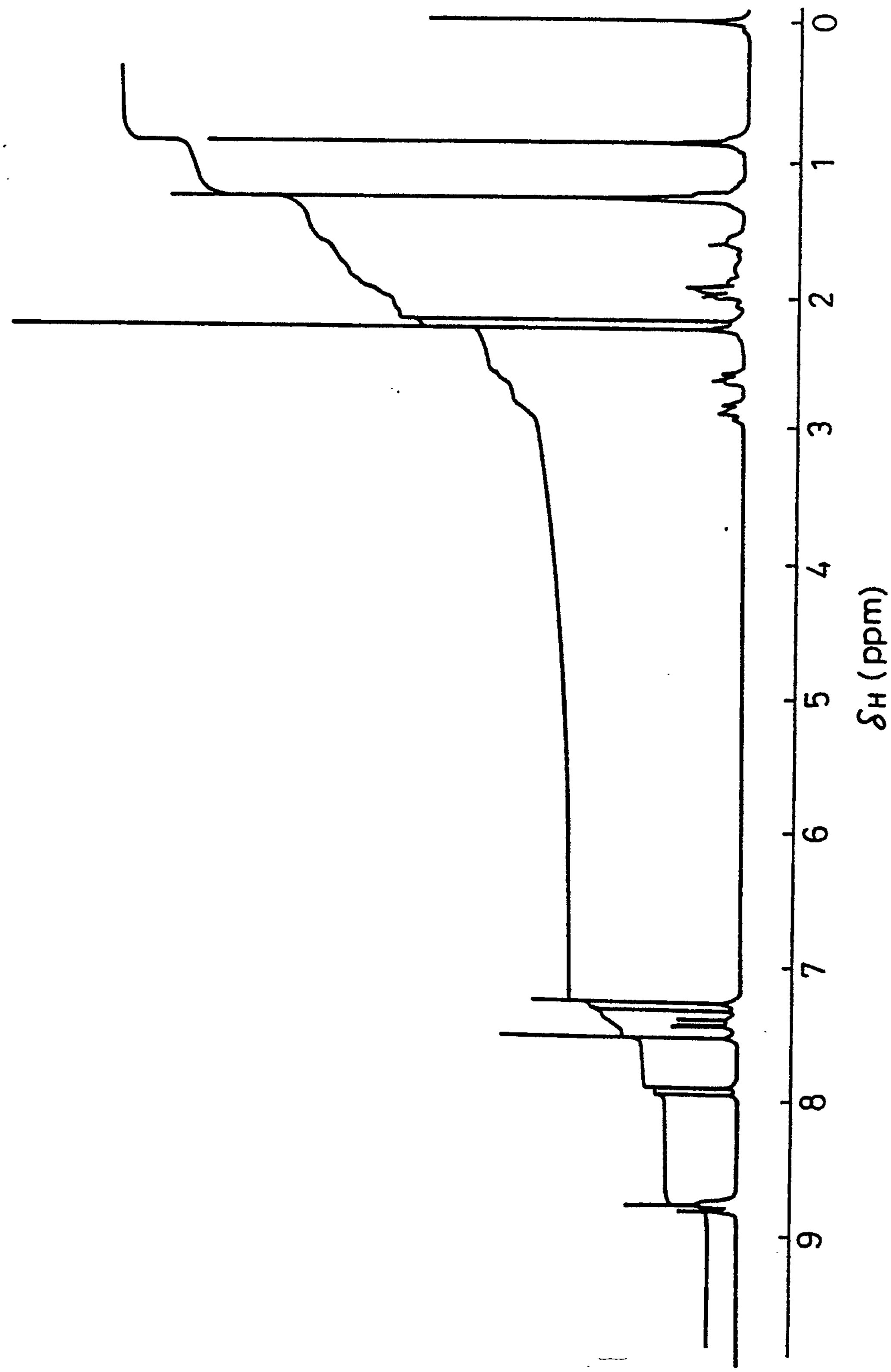

Fig. 2

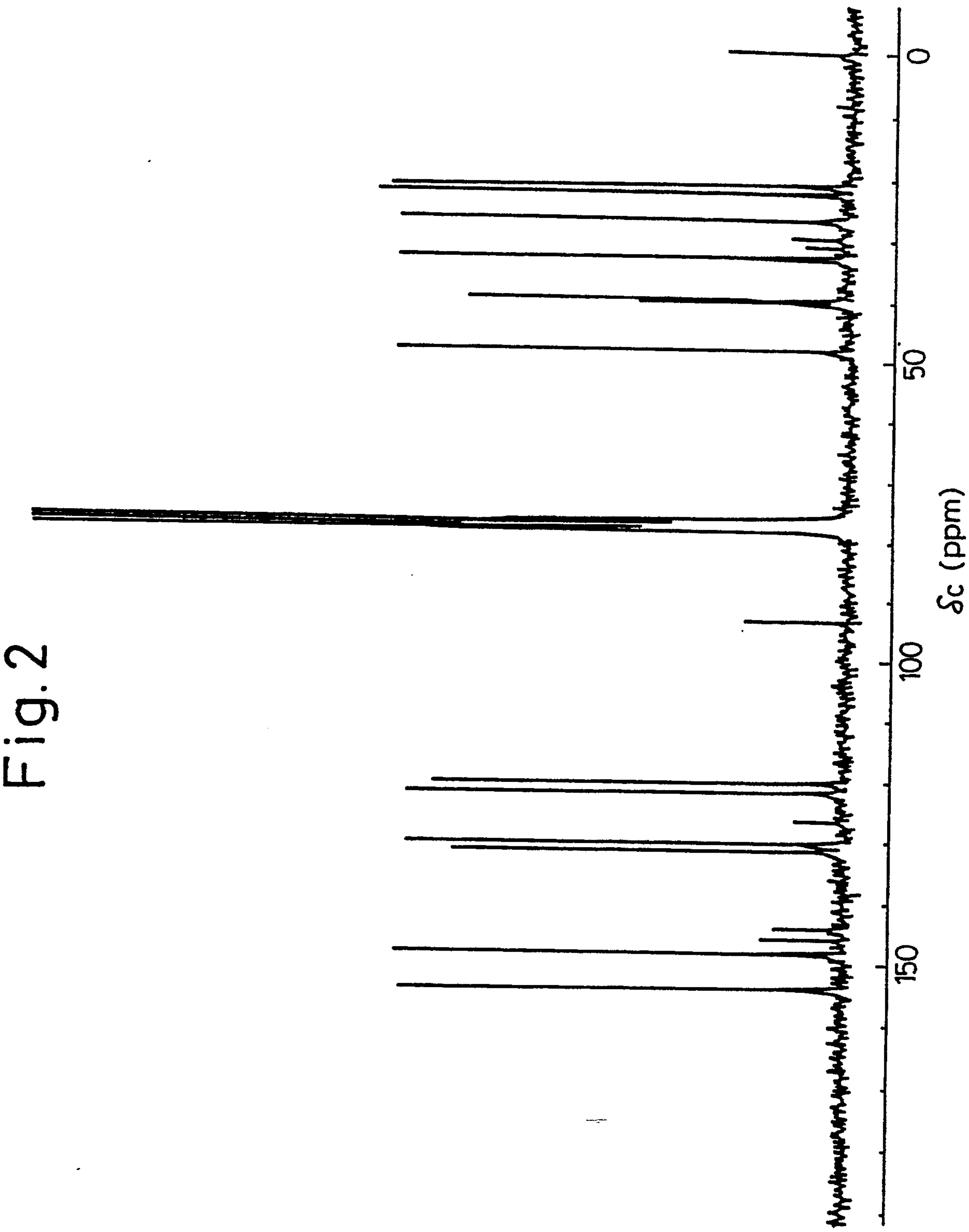

Fig. 3

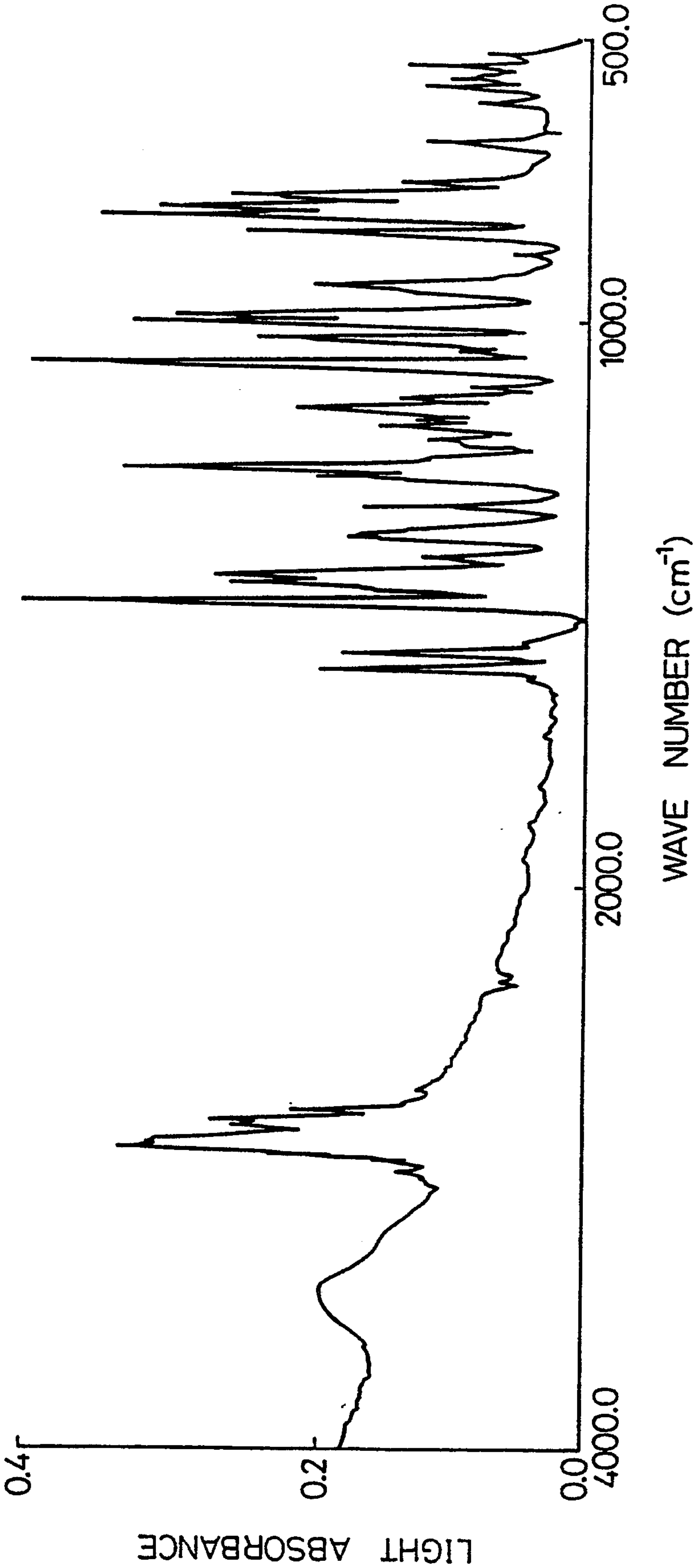
LIGHT ABSORBANCE
0.4
0.2
0.0
4000.0
2000.0
1000.0
500.0
WAVE NUMBER ($cm^{-1}$)

Fig. 4

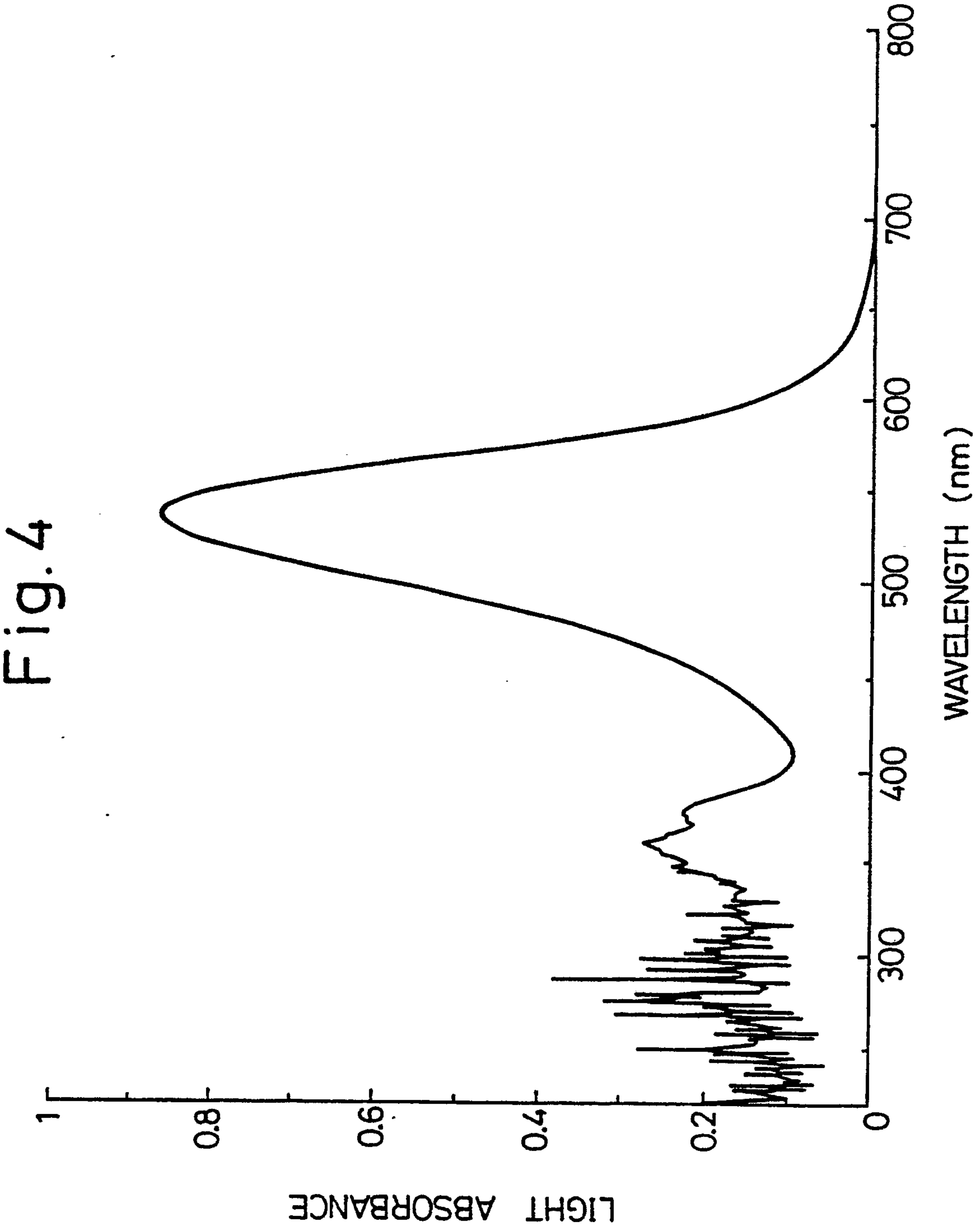
LIGHT ABSORBANCE
0
0.2
0.4
0.6
0.8
1
WAVELENGTH (nm)
300
400
500
600
700
800

Fig. 5

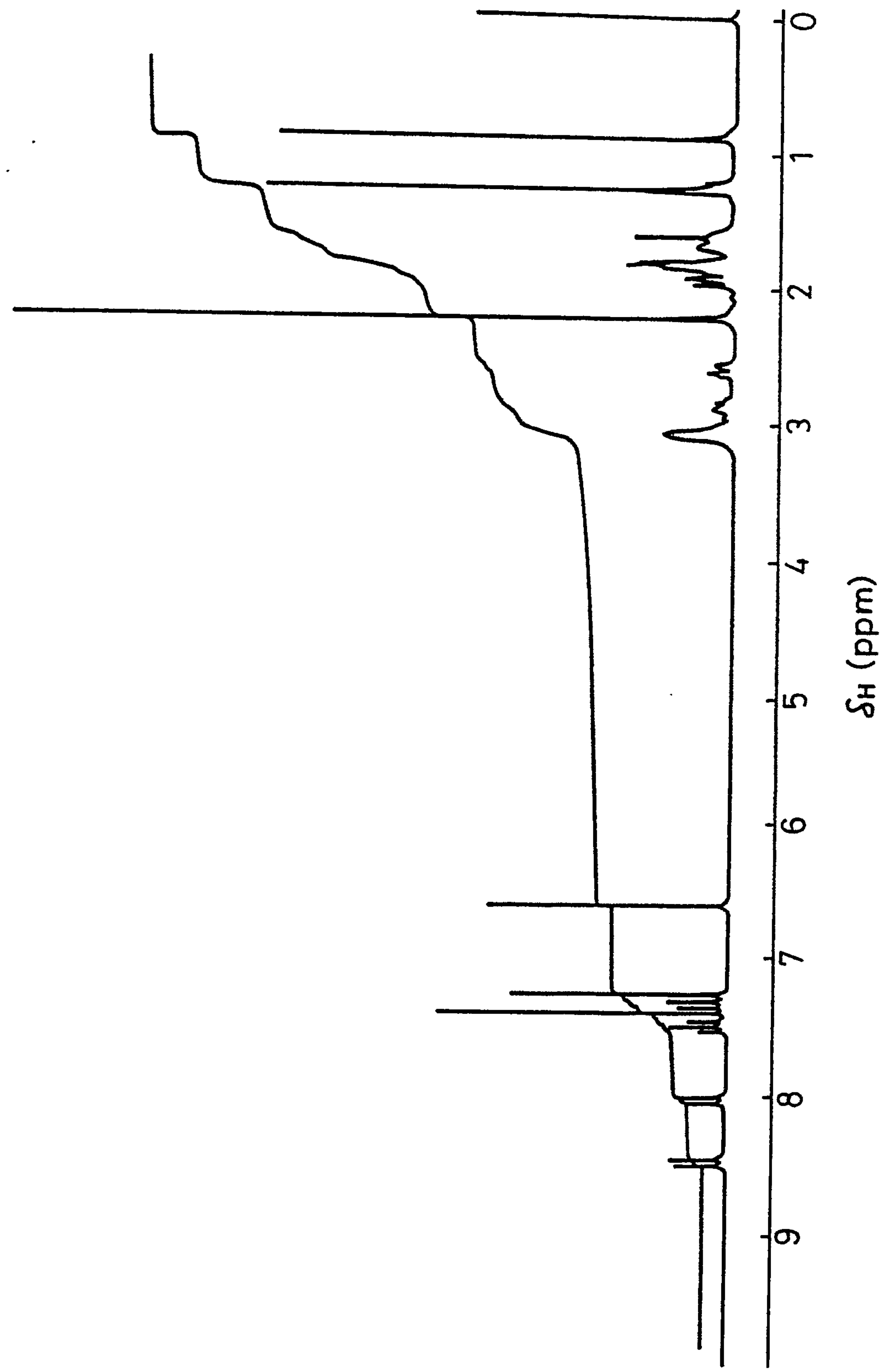

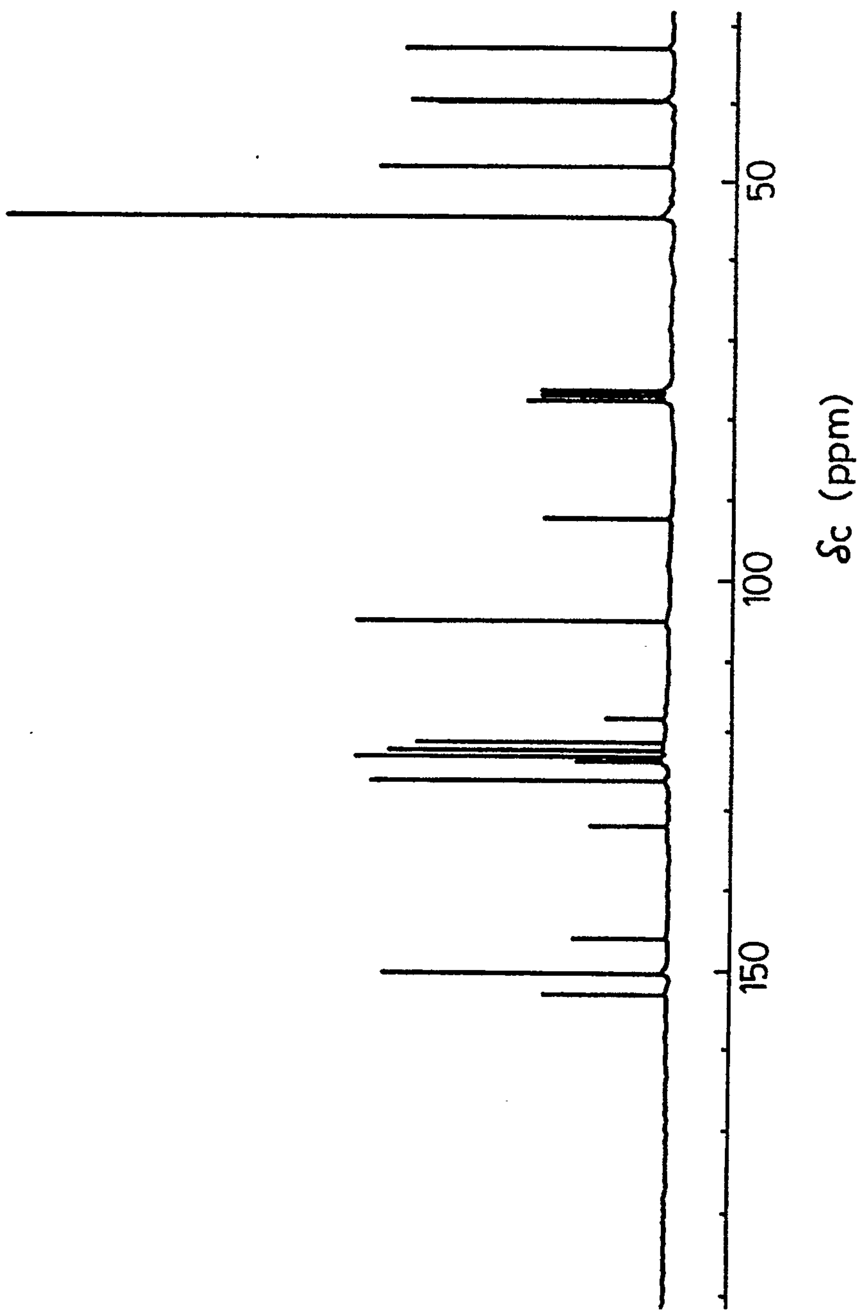


Fig. 6

Fig.7

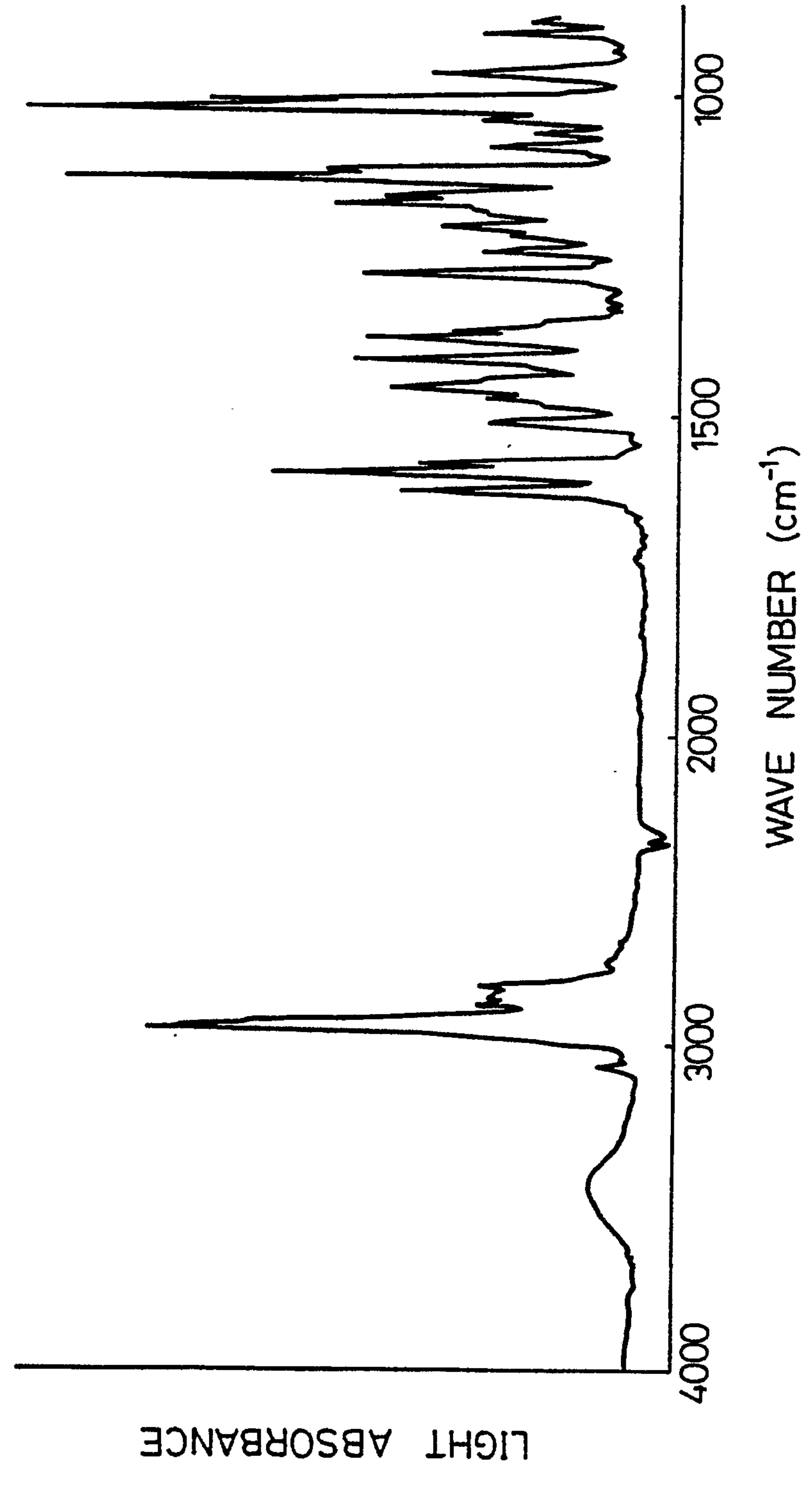

Fig. 8